# EUROPEAN PATENT APPLICATION

(11) **EP 1 415 651 A1**
(43) Date of publication of application: **06.05.2004**
(21) Application number: 02755819.6
(22) Date of filing: 05.08.2002
(51) Int. Cl.: A61K 31/4355, A61P 21/04, A61P 25/00, A61P 25/14, A61P 25/16, A61P 25/20, A61P 25/28, A61P 27/06, A61P 43/00, C07D 495/04, C07D 491/048

(54) **PREVENTIVES/REMEDIES FOR CHOLINERGIC NEUROPATHY**

(30) Priority: 06.08.2001 JP 2001238476
(71) Applicant: Mitsubishi Pharma Corporation, Osaka-shi, Osaka 541-0046 (JP)
(72) Inventor: TAKASHINA, Ken, MITSUBISHI PHARMA CORPORATION, Chuo-ku, Tokyo 103-8405 (JP); BESSHO, Tomoko, MITSUBISHI PHARMA CORPORATION, Chuo-ku, Tokyo 103-8405 (JP)
(74) Representative: Weber, Thomas, Dr.Dipl.-Chem.
(86) International application number: PCT/JP2002/007960
(87) International publication number: WO 2003/013522

(57) **Abstract**

An agent for prophylaxis or therapy of cholinergic nerve disorder, which contains, as an active ingredient, a pharmaceutical agent capable of increasing the choline transporter number on the cell membrane surface of cholinergic nerve terminal or a pharmaceutical agent capable of increasing the choline transporter number on the cell membrane surface by acting on intracellular localized changes of the choline transporters.

Provision of a pharmaceutical agent is possible, which does not require consideration of half-life in blood but affords dramatic administration frequency of two times or less a day in consideration of the mechanism of efficacy. This means improved compliance of patients in need of the treatment with the pharmaceutical agent and reduced burden on the caregiver of the patient.

## Description

### Technical Field

The present invention relates to an agent for the prophylaxis or therapy of cholinergic nerve disorders, and particularly relates to an agent for the prophylaxis or therapy of cholinergic nerve disorders, which contains, as an active ingredient, a pharmaceutical agent capable of increasing the choline transporter number on a cell membrane of a cholinergic nerve terminal.

### Background Art

With the hope of achieving an improving effect on the symptoms of dementia, which are considered to be attributable to lower cholinergic nerve functions as seen in the diseases such as Alzheimer's disease, Huntington's disease, Pick' disease, Down's syndrome, Parkinson's disease and the like, and further of achieving an improving effect on tardive dyskinesia, myasthenia gravis, glaucoma, dysgryphia and the like, some acetylcholine esterase inhibitors have been marketed in recent years and put to clinical use. The acetylcholine esterase inhibitors show efficacy by suppressing the decomposition of acetylcholine released from the acetylcholinergic nerve terminal, thereby increasing the concentration of acetylcholine in synaptic cleft. These pharmaceutical agents have been commonly reported to cause clinical side effects due to excessive activation of acetylcholinergic nerve even in non-targeted organs, such as vomition, nausea, gastrointestinal symptoms and the like. In an attempt to decrease expression of such side effects, a clinical administration method involving a titration period, wherein a dose is varied progressively from low dose to optimal efficacy dose, has been employed. The titration period accompanies variations in the therapeutic dose, thus lowering the compliance of medication, and means that the efficacy of a pharmaceutical agent is essentially associated with the occurrence of side effects. This may also mean that the setting of such titration period is a side effect caused by hyperstimulation of the acetylcholinergic nerve. The problem of side effects and the control of administration dose force a great burden on patients as well as on caregivers, and there is a great demand on a pharmaceutical agent that causes little side effect and is easy to take.

In the meantime, 4-acylamino-5,6,7,8-tetrahydrofuro[2,3-b]quinoline derivatives represented by the formula (Ib) to be mentioned below, wherein each symbol is as defined below, have been developed as a pharmaceutical agent that ameliorates symptoms of, for example, dementia seemingly attributable to lower cholinergic nerve functions as seen in the aforementioned diseases.

Choline is a substrate used for acetylcholine synthesis, and it is widely known that the rate-limiting step in acetylcholine synthesis is a high-affinity choline uptake (HACU). The above-mentioned 4-acylamino-5,6,7,8-tetrahydrofuro[2,3-b]quinoline derivatives have been reported to enhance high-affinity choline uptake in acetylcholinergic nerve terminal (JP-A-8-104633, Bessho T. et al., Effect of the novel high affinity choline uptake enhancer 2-(2-Oxo-pyrrolidin-1-yl)-N-(2,3-dimethyl-5,6,7,8-tetrahydrofuro[2,3-b]quinolin-4-yl)acetoamide on deficits of water mazelearning in rats. Arzneimittel-Forschung/Drug Research 46(1), 4, 369-373 (1996)). On the other hand, the above-mentioned compound has been reported to show a cell death suppressive effect by pre-ischemia administration in a rat transient cerebral ischemic model (JP-A-8-92100), and also clarified to have a nerve cell protecting effect. In addition, while no efficacy was shown by a single administration of the above-mentioned compound in a passive avoidance response test using a basal forebrain (BF) destruction model, which is a cholinergic nervous system disorder model, consecutive administration first showed an improving effect and this action was confirmed even 24 hr after the final administration. A single administration of the above-mentioned compound failed to show efficacy and the action mechanism in the BF destruction model is unknown at the moment. However, it is known that, generally in a BF destruction model, after preparing the disorder model, a nerve having a lower level of disorder in the vicinity of the disorder area is compensatorily activated and gradually recovers from the disorder, and that this recovery process is promoted by a neurotrophic factor and a neuroactive drug such as nootropic. In the case of the above-mentioned compound, too, the recovery process from the disorder was considered to have been promoted because recovery of acetylcholine content was observed in an administration group (Bessho T. et al., Effects of repeated administration of MKC-231 a novel choline uptake enhancer, on cholinergic deficits in basal forebrain-lesioned rats. Jap. J. Pharmacol. 73, Suppl., 173 (1997)). These non-clinical efficacy studies suggest the possibility of the above-mentioned compound having a function improving action that activates cholinergic nerve based on promotion of choline uptake, as well as cell disorder protecting action that promotes suppression of denatured cell death and recovery of cell disorder.

Inasmuch as a non-clinical efficacy study model reflects a certain aspect of a disease, logical inference of the clinical application method and dose from the test results essentially requires a pharmacokinetic understanding and understanding of the action mechanism of the drug. From the studies to the present, however, the action of the above-mentioned compound is clear but the site and mode of action are not known. For clinical application of this drug having a short blood half-life of about 0.7 hr, therefore, it is generally understood that the administration frequency to patients needs to be set to at least 3 times a day to maintain effective blood concentration as long as possible. The administration frequency of twice a day or once a day by increasing the single dose is considered to be practically impossible due to the risk of causing side effects and the like.

It is therefore an object of the present invention to provide an agent for the prophylaxis or therapy of various neurological disorders caused by lower cholinergic nerve functions, which agent is free of titration period, causes less side effects and permits easy control of doses. Another object of the present invention is to provide a method of screening a compound useful for the prophylaxis and/or therapy of cholinergic nerve disorders, which is based on a new mode of action.

### Disclosure of the Invention

The present inventors have conducted intensive studies noting the behavior of choline transporters in view of the above-mentioned objects, and found that HACU can be improved significantly and continuously by increasing the choline transporter number on a cell membrane, particularly by acting on intracellular localized changes of choline transporters, thereby to increase the choline transporter number on a cell membrane, and obtained a specific compound having such an action mechanism, which resulted in the completion of the present invention. In addition, by the elucidation of such action mechanism, administration of not more than twice a day, which has been considered to be unattainable heretofore, has been enabled. Accordingly, the present invention provides the following.
[1] A pharmaceutical agent for increasing the choline transporter number on the cell membrane surface of a cholinergic nerve terminal, which agent comprises, as an active ingredient, a compound of the formula (I) 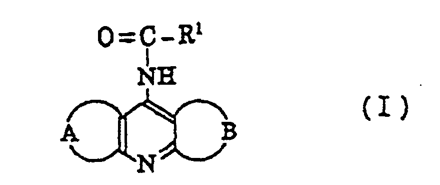 wherein R¹ is C₂-C₆ alkyl or the formula (II) 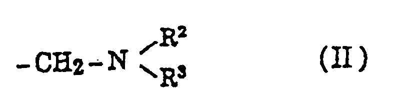 wherein R² is hydrogen atom or acetyl and R³ is C₁-C₆ alkyl, cycloalkyl or 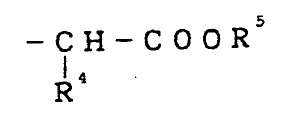 wherein R⁴ and R⁵ are each independently hydrogen atom or C₁-C₆ alkyl, and in 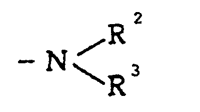 of the formula (II), R² and R³ may in combination form 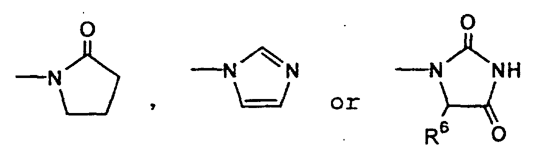 wherein R⁶ is hydrogen atom or C₁-C₆ alkyl; 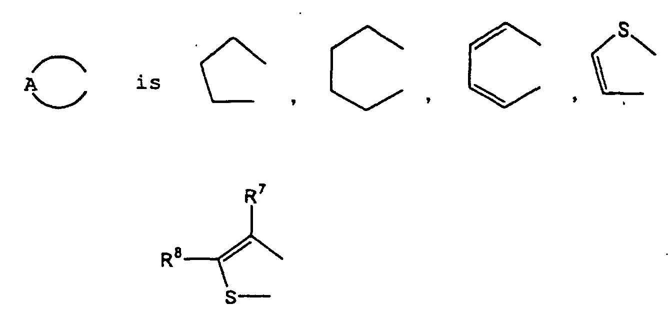 wherein R⁷ and R⁸ are each independently hydrogen atom or C₁-C₄ alkyl, 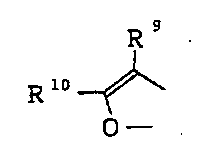 wherein R⁹ and R¹⁰ are each independently hydrogen atom or C₁-C₄ alkyl, 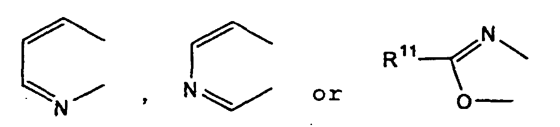 wherein R¹¹ is hydrogen atom or C₁-C₄ alkyl; and 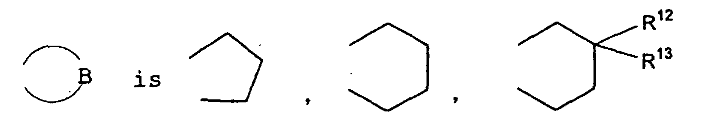 wherein R¹² and R¹³ are each independently C₁-C₄ alkyl or may in combination form 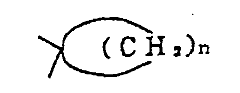 wherein n is an integer of 2 to 6, or 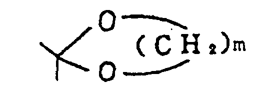 wherein m is an integer of 2 or 3, 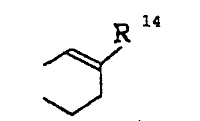 wherein R¹⁴ is hydrogen atom or C₁-C₄ alkyl, 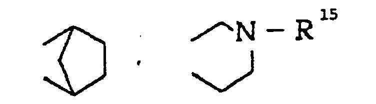 wherein R¹⁵ is hydrogen atom or aralkyl, or 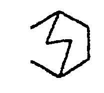 provided that when 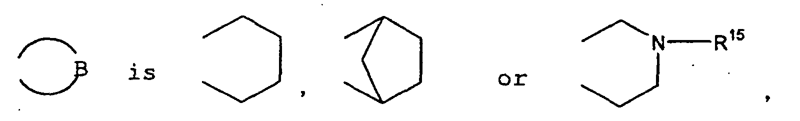 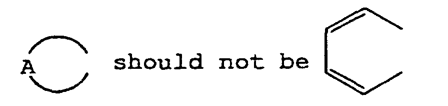 and R⁷ should not be hydrogen atom, optical enantiomers thereof, an acid addition salt thereof or a hydrate or solvate thereof.
[2] The pharmaceutical agent of the above-mentioned [1], wherein the compound of the formula (I) is a compound of the formula (Ia) 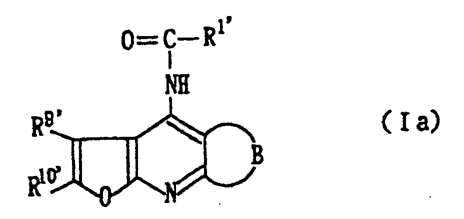 wherein R¹' is C₂-C₆ alkyl or the formula (II)' 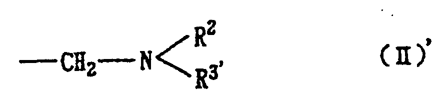 wherein R² is hydrogen atom or acetyl, and R³' is C₁-C₆ alkyl or 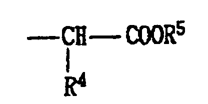 wherein R⁴ and R⁵ are each independently hydrogen atom or C₁-C₆ alkyl, and in 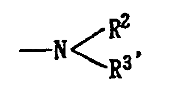 of the formula (II)', R² and R³' may in combination form 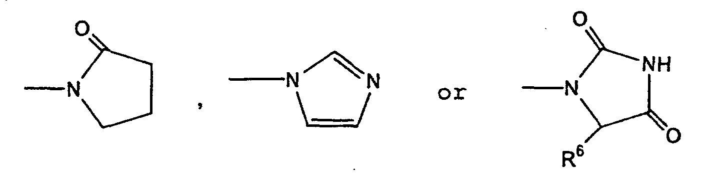 wherein R⁶ is hydrogen atom or C₁-C₆ alkyl;
   R⁹' and R¹⁰' are each independently C₁-C₄ alkyl; and 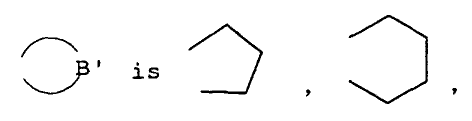 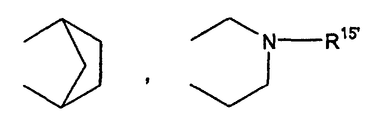 wherein R¹⁵' is aralkyl, or 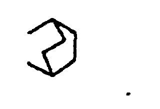
[3] The pharmaceutical agent of the above-mentioned [1], wherein the compound of the formula (I) is a 4-acylamino-5,6,7,8-tetrahydrofuro[2,3-b]quinoline derivative of the formula (Ib) 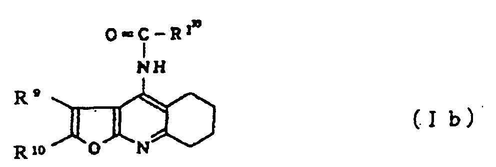 wherein R¹'' is C₂-C₆ alkyl, 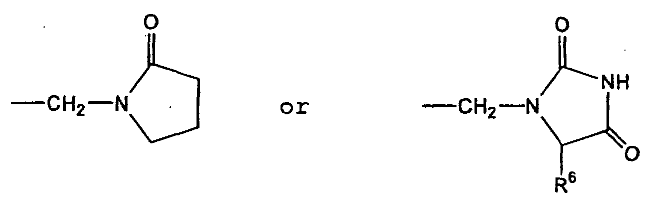 wherein R⁶ is hydrogen atom or C₁-C₆ alkyl; and
   R⁹ and R¹⁰ are each independently hydrogen atom or C₁-C₄ alkyl.
[4] The pharmaceutical agent of the above-mentioned [1], wherein the compound of the formula (I) is 2-(2-oxopyrrolidin-1-yl)-N-(2,3-dimethyl-5,6,7,8-tetrahydrofuro[2,3-b]quinolin-4-yl)acetamide.
[5] A pharmaceutical agent for increasing the choline transporter number on the cell membrane surface by acting on intracellular localized changes of the choline transporters, which agent comprises, as an active ingredient, a compound of the formula (I), optical enantiomers thereof, an acid addition salt thereof or a hydrate or solvate thereof.
[6] The pharmaceutical agent of the above-mentioned [5], wherein the compound of the formula (I) is a compound of the formula (Ia).
[7] The pharmaceutical agent of the above-mentioned [5], wherein the compound of the formula (I) is a compound of the formula (Ib).
[8] The pharmaceutical agent of the above-mentioned [5], wherein the compound of the formula (I) is 2-(2-oxopyrrolidin-1-yl)-N-(2,3-dimethyl-5,6,7,8-tetrahydrofuro[2,3-b]quinolin-4-yl)acetamide.
[9] An agent for the prophylaxis or therapy of a cholinergic nerve disorder, which comprises, as an active ingredient, a pharmaceutical agent that increases the choline transporter number on the cell membrane surface of the cholinergic nerve terminal.
[10] The prophylactic or therapeutic agent of the above-mentioned [9], wherein the pharmaceutical agent is the agent of any of the above-mentioned [1] to [4].
[11] An agent for the prophylaxis or therapy of a cholinergic nerve disorder, which comprises, as an active ingredient, a pharmaceutical agent that acts on intracellular localized changes of choline transporters to increase the choline transporter number on the cell membrane surface.
[12] The prophylactic or therapeutic agent of the above-mentioned [11], wherein the pharmaceutical agent is the agent of any of the above-mentioned [5] to [8].
[13] The prophylactic or therapeutic agent of any of the above-mentioned [9] to [12], wherein the cholinergic nerve disorder is dementia caused by Alzheimer's disease, Huntington's disease, Pick' disease, Down's syndrome or Parkinson's disease, tardive dyskinesia, myasthenia gravis, glaucoma or dysgryphia.
[14] The prophylactic or therapeutic agent of any of the above-mentioned [9] to [13], which is administered not more than twice a day.
[15] The prophylactic or therapeutic agent of any of the above-mentioned [9] to [14], which causes reduced side effects due to hyperstimulation of acetylcholinergic nerve.
[16] A commercial package comprising a pharmaceutical agent of any of the above-mentioned [1] to [4] and a written matter associated therewith, the written matter stating that the pharmaceutical agent can or should be used for the prophylaxis and/or therapy of a cholinergic nerve disorder.
[17] A commercial package comprising a pharmaceutical agent of any of the above-mentioned [5] to [8] and a written matter associated therewith, the written matter stating that the pharmaceutical agent can or should be used for the prophylaxis and/or therapy of a cholinergic nerve disorder.
[18] The commercial package of the above-mentioned [16] or [17], wherein the written matter states that the pharmaceutical agent is administered not more than twice a day.
[19] A method of screening a compound useful for the prophylaxis or therapy of a cholinergic nerve disorder, which comprises at least the steps of
   (1) bringing a test compound into contact with a cell membrane of a cholinergic nerve terminal, and
   (2) counting the transporter number on a surface of the cell membrane.
[20] A pharmaceutical agent for the prophylaxis or therapy of cholinergic nerve disorder, which comprises, as an active ingredient, a compound of the formula (I), optical enantiomers thereof, an acid addition salt thereof or a hydrate or solvate thereof, wherein the agent is administered not more than twice a day.
[21] The prophylactic or therapeutic agent of the above-mentioned [20], wherein the compound of the formula (I) is a compound of the formula (Ia).
[22] The prophylactic or therapeutic agent of the above-mentioned [20], wherein the compound of the formula (I) is a 4-acylamino-5,6,7,8-tetrahydrofuro[2,3-b]quinoline derivative of the formula (Ib).
[23] The prophylactic or therapeutic agent of the above-mentioned [20], wherein the compound of the formula (I) is 2-(2-oxopyrrolidin-1-yl)-N-(2,3-dimethyl-5,6,7,8-tetrahydrofuro[2,3-b]quinolin-4-yl)acetamide of the formula (I).
[24] The prophylactic or therapeutic agent of any of the above-mentioned [20] to [23], wherein the cholinergic nerve disorder is dementia caused by Alzheimer's disease, Huntington's disease, Pick' disease, Down's syndrome or Parkinson's disease, tardive dyskinesia, myasthenia gravis, glaucoma or dysgryphia.
[25] The prophylactic or therapeutic agent of any of the above-mentioned [20] to [23], wherein the cholinergic nerve disorder is dementia caused by Alzheimer's disease, tardive dyskinesia, myasthenia gravis, glaucoma or dysgryphia.
[26] The prophylactic or therapeutic agent of any of the above-mentioned [20] to [25], which is administered once a day.

### Brief Description of the Drawings

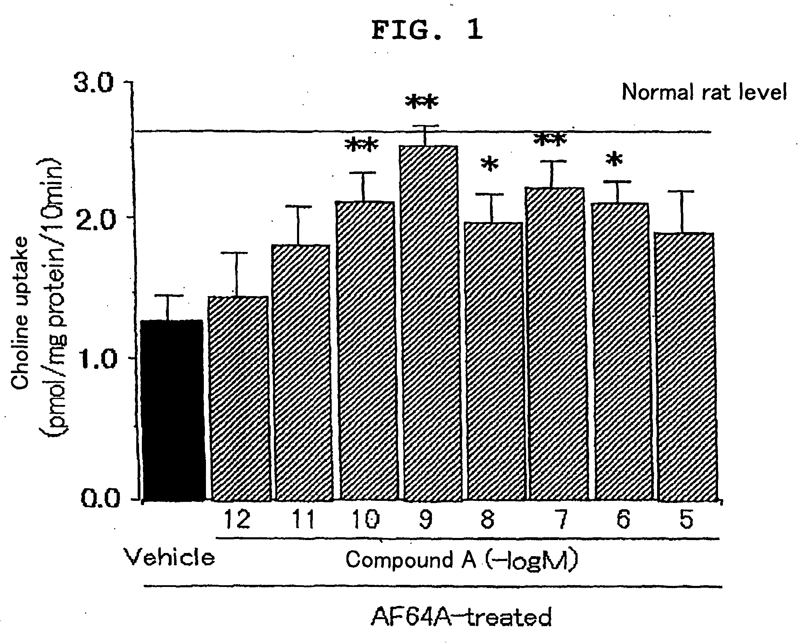

Fig. 1 is a graph showing choline uptake in Example 1, wherein the axis of abscissas shows the concentration of Compound A, the axis of ordinates shows the high-affinity choline uptake, ** shows p<0.01 and * shows p<0.05 (vs Vehicle, Dunnett's test).

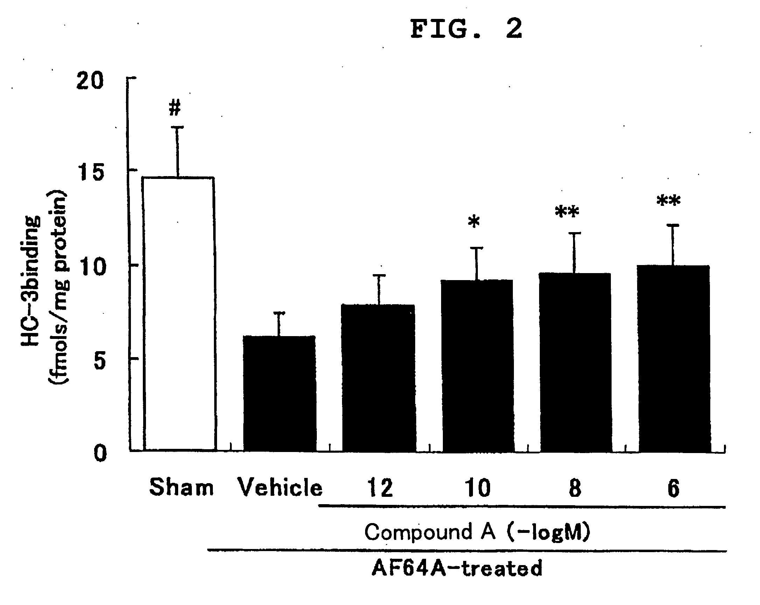

Fig. 2 is a graph showing the level of hemicholinium-3 binding in Example 2, wherein the axis of abscissas shows the presence or otherwise of AF64A treatment and concentration of Compound A, the axis of ordinates shows the amount of binding of hemicholinium-3, which is a specific ligand of choline transporter, # shows p<0.05 (vs Vehicle, t-test), ** shows p<0.01 and * shows p<0.05 (vs Vehicle, Dunnett's test).

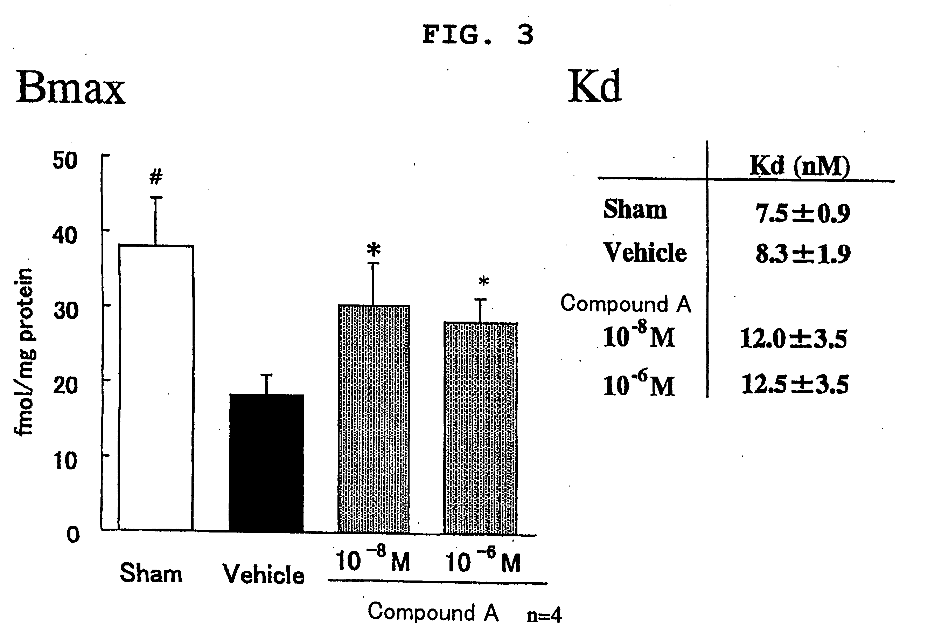

Fig. 3 shows the maximum amount of binding (Bmax) and dissociation constant (Kd) in Example 2, relative to the binding of hemicholinium-3, wherein the axis of abscissas of the graph of Bmax shows the presence or otherwise of AF64A treatment and concentration of Compound A, the axis of ordinates shows the maximum amount of binding of hemicholinium-3, # shows p<0.01 (vs Vehicle, t-test) and * shows p<0.05 (vs Vehicle, Dunnett's test).

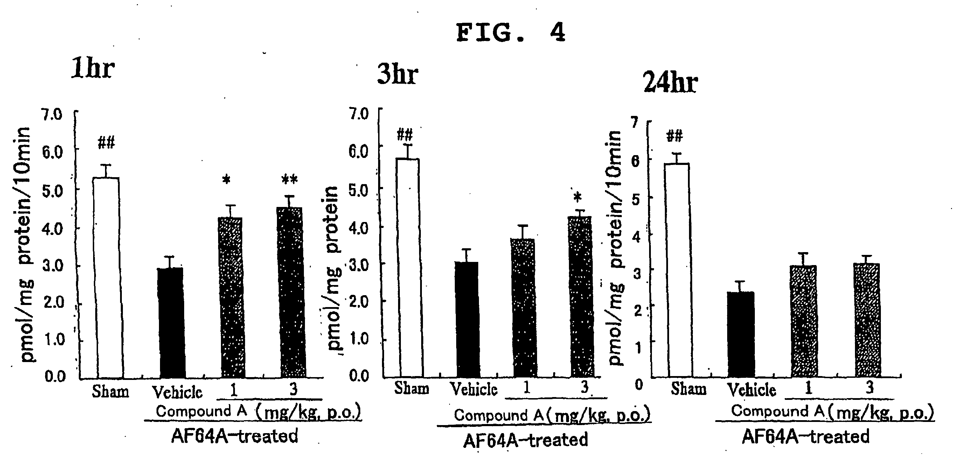

Fig. 4 includes graphs showing time-course changes in choline uptake after single administration of Compound A in Example 3, which was determined at 1 h, 3 h and 24 h after the single administration, wherein the axis of abscissas of each graph shows the presence or otherwise of AF64A treatment and concentration of Compound A, the axis of ordinates shows the high-affinity choline uptake, ## shows p<0.01 (vs Vehicle, t-test), ** shows p<0.01 and * shows p<0.05 (vs Vehicle, Dunnett's test).

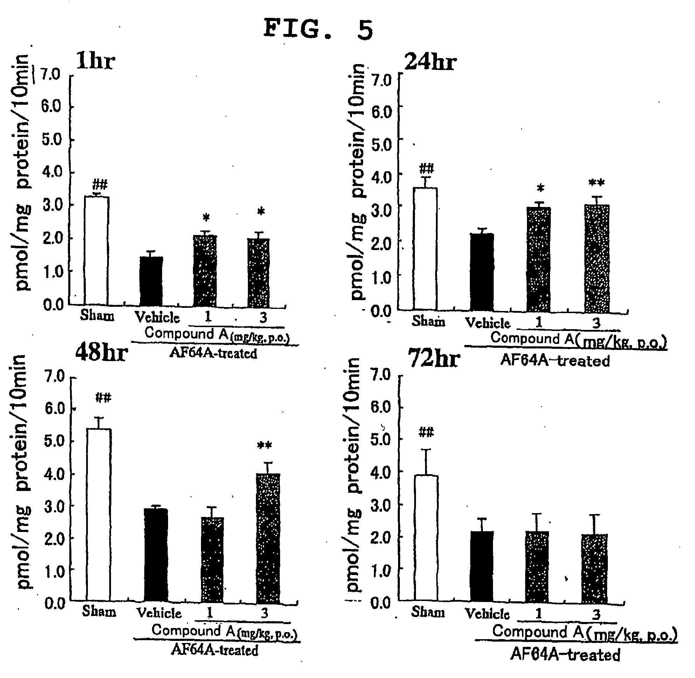

Fig. 5 includes graphs showing time-course changes in choline uptake, after administration for 8 consecutive days of Compound A in Example 3, which was determined at each passage point in time at 1 h, 24 h, 48 h and 72 h after the single administration, wherein the axis of abscissas of each graph shows the presence or otherwise of AF64A treatment, concentration of Compound A, the axis of ordinates shows the high-affinity choline uptake, ## shows p<0.01 (vs Vehicle, t-test), ** shows p<0.01 and * shows p<0.05 (vs Vehicle, Dunnett's test).

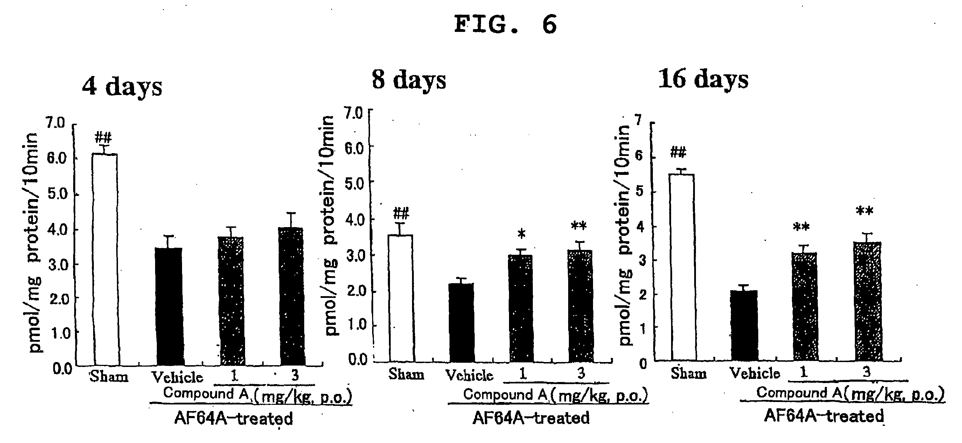

Fig. 6 includes graphs showing choline uptake, after administration for 4, 8 and 16 consecutive days of Compound A and a lapse of 24 hr in Example 3, wherein the axis of abscissas of each graph shows the presence or otherwise of AF64A treatment and concentration of Compound A, the axis of ordinates shows the high-affinity choline uptake, ## shows p<0.01 (vs Vehicle, t-test), ** shows p<0.01 and * shows p<0.05 (vs Vehicle, Dunnett's test).

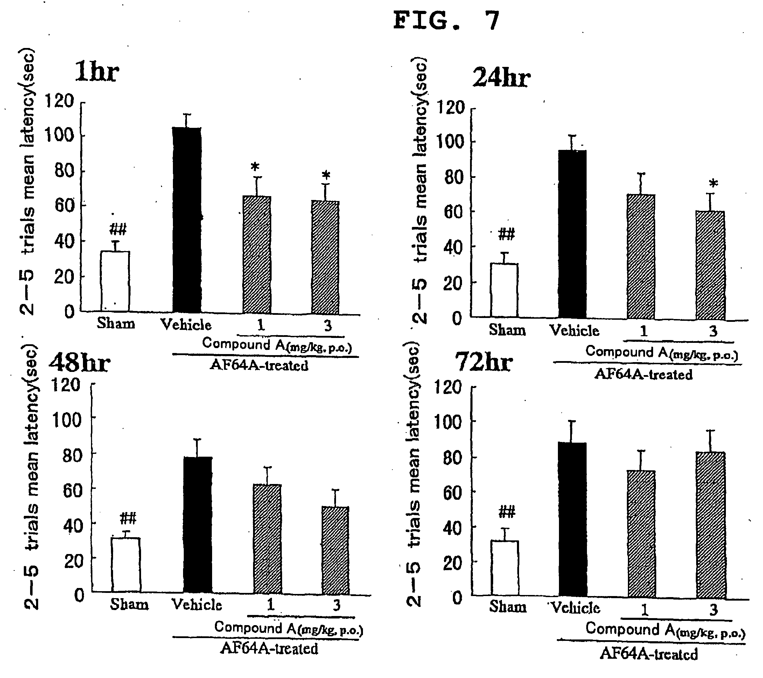

Fig. 7 includes graphs showing a time-course water maze-learning disorder improving effect measured at each passage point in time after administration for 8 consecutive days of Compound A in Example 3, which was determined at each passage point in time at 1 h, 24 h, 48 h and 72 h after the consecutive administration, wherein the axis of abscissas of each graph shows the presence or otherwise of AF64A treatment and concentration of Compound A, the axis of ordinates shows the average time necessary for arrival (2 to 5 times of trial), ## shows p<0.01 (vs Vehicle, t-test) and * shows p<0.05 (vs Vehicle, Dunnett's test).

### Best Mode for Embodying the Invention

The present invention is described in detail in the following. The C₂-C₆ alkyl at R¹, R¹' and R¹" in the present invention is preferably C₂-C₄ alkyl, such as ethyl, n-propyl, isopropyl, n-butyl, sec-butyl, tert-butyl and the like.

The C₁-C₆ alkyl at R³ and R³' is preferably C₁-C₄ alkyl, such as methyl, ethyl, n-propyl, isopropyl, n-butyl, sec-butyl, tert-butyl and the like. The cycloalkyl at R³ is preferably C₃-C₆ cycloalkyl, such as cyclopropyl, cyclobutyl, cyclopentyl, cyclohexyl and the like.

The C₁-C₆ alkyl at R⁴-R⁶ is preferably C₁-C₄ alkyl, such as methyl, ethyl, n-propyl, isopropyl, n-butyl, sec-butyl, tert-butyl and the like.

The C₁-C₄ alkyl at R⁷-R¹⁴, R⁹' and R¹⁰' is preferably C₁-C₄ alkyl, such as methyl ethyl, n-propyl, isopropyl, n-butyl, sec-butyl, tert-butyl and the like.

The aralkyl at R¹⁵ and R¹⁵' is phenyl substituted with C₁-C₄ alkyl, such as benzyl, phenethyl and the like.

In the present invention, the acid to form an acid addition salt of a compound of the formula (I) [encompassing the compounds of the formula (Ia) and the formula (Ib), hereinafter these compounds are also generally referred to conveniently as a compound of the formula (I)] includes, for example, inorganic acids such as hydrochloric acid, hydrobromic acid, hydriodic acid, sulfuric acid, phosphoric acid and the like, and organic acids such as oxalic acid, maleic acid, fumaric acid, lactic acid, malic acid, citric acid, tartaric acid, benzoic acid, methanesulfonic acid, camphersulfonic acid and the like. The acid addition salt to be administered should be acceptable as a pharmaceutical agent.

The above-mentioned compound of the formula (I) and an acid addition salt thereof may be in the form of a hydrate or solvate and these hydrates and solvates are also encompassed in the compound that is an active ingredient of the present invention. The above-mentioned compound of the formula (I) may have an optical enantiomer, which is also encompassed in the compound that is an active ingredient of the present invention. The production method of the compounds of the formula (I) and the like, which are contained in the pharmaceutical agents and medicaments of the present invention as an active ingredient, is not particularly limited. For example, they can be synthesized easily according to the method described in JP-A-3-218361 (JP patent No. 2546919) and appropriately using the method known in the pertinent field.

The above-mentioned compound of the formula (I), particularly a compound of the formula (Ia), particularly preferably a compound of the formula (Ib), i.e., 4-acylamino-5,6,7,8-tetrahydrofuro[2,3-b]quinoline derivative and the like, remarkably increase the choline transporter number on the cell membrane surface of a cholinergic nerve terminal, as mentioned in examples to be mentioned below, and remarkably increase the number by particularly acting on intracellular localized changes (hereinafter to be also referred to as translocation) of the choline transporter by promoting transfer of the transporters to the cell membrane surface. By increasing the transporter number on the cell membrane surface, the choline uptake can be promoted and the cholinergic nerve is activated. Therefore, 4-acylamino-5,6,7,8-tetrahydrofuro[2,3-b]quinoline derivative and the like of the above-mentioned formula (Ib) has an improving action on various cholinergic nerve disorders and can be used as an agent for the therapy or prophylaxis of dementia caused by Alzheimer's disease, Huntington's disease, Pick' disease, Down's syndrome, Parkinson's disease and the like. Moreover, this derivative can be used as an agent for the therapy or prophylaxis of tardive dyskinesia, myasthenia gravis, glaucoma, dysgryphia and the like.

The choline transporter is a substance responsible for the choline uptake. The transporter has been identified one after another in rat and human in recent years and clarified to have twelve transmembrane domains and a homology with a glucose transporter family (rat: Nature Neuroscience No. 3, pp. 120-125, 2000, human: FEBS Letters vol. 484, pp. 92-97, 2000, Biochemical and Biophysical Research Communications vol. 276, pp. 862-867, 2000).

In the context of the present invention, the cell membrane of cholinergic nerve terminal is not particularly limited as long as it expresses a choline transporter. It is preferably a cell membrane derived from hippocampus, which is used in the examples to be mentioned below.

Based on the action mechanism wherein a high-affinity choline uptake (HACU) is promoted by increasing the choline transporter number on the cell membrane surface, whereby the cholinergic nerve is activated, the present invention provides a pharmaceutical agent capable of increasing the number of choline transporters on the cell membrane surface of a cholinergic nerve terminal, and a pharmaceutical agent capable of increasing the number thereof on the cell membrane surface by acting on intracellular localized changes of the choline transporter. Such pharmaceutical agent is exemplified by one preferably containing the aforementioned 4-acylamino-5,6,7,8-tetrahydrofuro[2,3-b]quinoline derivative of the formula (Ib) as an active ingredient. The present invention further provides a new method of screening a compound useful for the prophylaxis or therapy of cholinergic nerve disorders. The screening method of the present invention includes at least the steps of (1) bringing a test compound with a cell membrane of the cholinergic nerve terminal and (2) counting the transporters on the surface of the cell membrane, and judging the usefulness of the compound. The test compound here embraces any compound intended for the application to the prophylaxis or therapy of cholinergic nerve disorders, whether it is known or unknown. The cell membrane of the cholinergic nerve terminal to be prepared for the screening method of the present invention is not particularly limited as long as it is a membrane sample of a cell that expresses a choline transporter (see above), which is preferably hippocampal synaptosome. More preferably, it is a membrane sample prepared from an animal that has been administered in advance with a specific inhibitor (e.g., specific neurotoxin, AF64A) against the cholinergic nerve to lower HACU, so that the effect would be clearer. The preparation method of the membrane sample is known. The membrane sample is brought into contact with the test compound according to a method typically employed. For example, the membrane sample and the test compound are incubated in a suitable buffer, such as Ringer's solution and the like, at a suitable temperature for a suitable time period. The transporter number on the cell membrane surface can be counted by a method typically employed in the pertinent field or a combination of such methods. For example, a method wherein a labeled product of hemicholinium-3, which is a ligand specific to the choline transporter, is utilized, a method wherein an antibody specific to the choline transporter is prepared and a labeled product of the antibody is utilized, and the like.

When the compound of the above-mentioned formula (I), particularly 4-acylamino-5,6,7,8-tetrahydrofuro[2,3-b]quinoline derivative of the formula (Ib), and the like are used as a pharmaceutical agent, they are administered alone or in combination with a pharmacologically acceptable carrier. The composition is determined according to the solubility and chemical property of the compound, administration route, administration schedule and the like. For example, the compound may be processed into a dosage form such as granules, fine particles, powders, tablets, hard capsules, soft capsules, syrups, emulsions, suspensions, liquids and the like and orally administered. It may be prepared into an injection for intravenous, intramuscular administration or subcutaneous administration and administered.

The compound may be prepared into a powder for injection or may be prepared when in use. An organic or inorganic, solid or liquid carrier or diluent for pharmaceutical agents suitable for oral, transrectal, parenteral or local administration can be used together with the 4-acylamino-5,6,7,8-tetrahydrofuro[2,3-b]quinoline derivative of the above-mentioned formula (Ib) and the like. The excipient to be used for production of solid preparation includes, for example, lactose, sucrose, starch, talc, cellulose, dextrin, Kaolin, calcium carbonate and the like. Liquid preparations for oral administration such as emulsion, syrup, suspension, liquid and the like contain an inert diluent generally used, such as water, vegetable oil and the like. This preparation may contain, besides the inert diluent, auxiliary agents such as moistening agent, suspending agent, sweetener, flavoring agent, coloring agent, preservative and the like. It is also possible to prepare the derivative as a liquid preparation to be filled in a capsule made of an absorbable substance, such as gelatin. The solvent or suspending agent to be used for preparations for parenteral administration, namely, injection and the like, may be, for example, water, propylene glycol, polyethylene glycol, benzyl alcohol, ethyl oleate, lecithin and the like. The preparation method may be a conventional one.

The clinical dose in the case of oral administration is a daily dose of 1-2000 mg, preferably 1-500 mg, for an adult as an active ingredient compound, which is increased or decreased as appropriate according to age, symptom, disease state, and presence or otherwise of concurrent administration. The frequency of administration is once a day of a daily dose, or two or three times of administrations a day at suitable intervals, or intermittent administration. For administration by injection, a daily dose of 0.1-100 mg, preferably 0.1-50 mg, is given to an adult.

It has been clarified in the present invention that the compound of the above formula (I) can increase the choline transporter number on the surface of the cell membrane of cholinergic nerve terminal, particularly that it acts on the intracellular localized changes of the choline transporter to increase its number on the cell membrane surface, which in turn has clarified that the compound of the above formula (I) can be sufficiently administered not more than twice a day, preferably once a day.

The pharmaceutical agent of the present invention capable of increasing the choline transporter number on the cell membrane surface of a cholinergic nerve terminal, particularly a pharmaceutical agent capable of increasing the choline transporter number on the cell membrane surface by acting on the intracellular localized changes thereof, is packaged together with a written matter stating that the pharmaceutical agent can or should be used for the prophylaxis or therapy of cholinergic nerve disorders, whereby a pharmaceutical product is preferably provided.

### Examples

The present invention is explained in more detail in the following by way of Examples. The present invention is not limited by the following Examples as long as the invention is within the scope of the present invention. As the compound of the above-mentioned formula (I), 2-(2-oxopyrrolidin-1-yl)-N-(2,3-dimethyl-5,6,7,8-tetrahydrofuro[2,3-b]quinolin-4-yl)acetamide (hereinafter Compound A), which is one of the 4-acylamino-5,6,7,8-tetrahydrofuro[2,3-b]quinoline derivatives of the formula (Ib), was synthesized according to the method described in JP-A-3-218361 and subjected to the following experiments.

### Example 1: Effect on the high-affinity choline uptake

The action of the compound of the formula (I), particularly 4-acylamino-5,6,7,8-tetrahydrofuro[2,3-b]quinoline derivative, on the high-affinity choline uptake was examined using [³H]choline and hippocampal synaptosome (membrane sample). AF64A (3 µmol), which is a neurotoxin specific to the cholinergic nerve, was administered to male Wistar rats into the cerebral ventricles bilaterally. The rats were subjected to an experiment after a recovery period of about 10 days. Hippocampal membrane samples (P2 fraction) were prepared by a conventional method from these animals and the animals that underwent a sham operation. These membrane samples were incubated in Ringer's solution with Compound A at each concentration or a solvent (vehicle) at 37°C for 30 min. [³H] Choline was added to the reaction mixture, and the mixture was incubated for 10 min. After the completion of the incubation, the membrane samples were filtrated on a glass fiber filter using a cell harvester and washed three times with a buffer. After thorough drying, a scintillator was added and the radioactivity of the membrane samples was measured on a liquid scintillation counter. Non-specific uptake was done under ice-cooling.

The results are shown in Fig. 1. Compound A significantly improved the choline uptake of hippocampal membrane samples having reduced choline uptake due to the AF64A treatment. It has been reported that promoted high-affinity choline uptake can be observed after Ca²⁺-dependent acetylcholine release or depolarization induced by high K⁺ stimulation, but it is unknown if the promotion is based on the expression of a new transporter or derived from an increased activity of already existent respective transporters (Can. J. Physiol. Pharmacol. 1986). The improving action on choline uptake observed at this time was further analyzed in detail.

### Example 2: Effect on the binding of hemicholinium-3

The action of the compound of the formula (I), particularly 4-acylamino-5,6,7,8-tetrahydrofuro[2,3-b]quinoline derivative, relative to the density of the high-affinity choline transporter on the nerve terminal was examined using [³H]hemicholinium-3, which is a specific labeled ligand of a high-affinity choline transporter (SYNAPSE No. 1, pp. 293-303, 1987). The aforementioned AF64A-treated rats were used in this test, too. Hippocampal membrane samples (P2 fraction) were prepared according to a conventional method from the AF64A-treated animals and the sham operation animals. These membrane samples were incubated in Ringer's solution with Compound A at each concentration or a solvent (vehicle) at 37°C for 30 min. After the completion of the incubation, the drug was removed from the reaction mixture by centrifugation at 4°C and the membrane samples were washed. The drug-treated membrane samples thus obtained were suspended in a solution containing [³H] hemicholinium-3, and incubated overnight at 4°C. After the completion of the incubation, the membrane samples were filtrated on a glass fiber filter using a cell harvester and washed three times with a buffer. After thorough drying, a scintillator was added and the radioactivity of the membrane samples was measured on a liquid scintillation counter. The non-specific binding was done in the presence of 10 µM non-specific hemicholinium-3. For the analysis of binding mode, Scatchard-plot was used and the maximum binding Bmax and dissociation constant Kd were calculated.

The results are shown in Fig. 2 and Fig. 3. With the incubation in advance with 4-acylamino-5,6,7,8-tetrahydrofuro[2,3-b]quinoline derivative, the binding of [³H]hemicholinium-3 on the hippocampal membrane sample increased dose-dependently (Fig. 2). This binding was applied to the Scatchard-plot analysis. As a result, it was clarified that the action of 4-acylamino-5,6,7,8-tetrahydrofuro[2,3-b]quinoline derivative was not on dissociation constant Kd, which is an index reflecting the binding affinity, but an increasing action on the maximum binding Bmax, which is an index reflecting changes in the transporter number on the surface of the membrane (Fig. 3).

Recent studies on the functional control of transporters have been focused on the following two control mechanisms. One is a functional control involving phosphorylation of transporter itself, and activation and inactivation by structural changes and the like, wherein the importance of phosphorylated modification site on each transporter for the activity control of transporter has been noted. Choline transporter is also speculated to have a site that can be phosphorylated by protein kinase, such as PKC and PKA.

Another mechanism drawing attention is functional control by translocation. This refers to the activity control based on changes in the amount of transporters on the surface of the membrane wherein the transporters localized in an intracellular pool (transporter store) and membrane surface are made to come and go between them.

The presence of such mechanism has been suggested in GLT4 of glucose transporter family having an analog structure of a twelve transmembrane domain type, SERTs which is a 5-HT transporter of the amine transporter family, GAT1 which is a GABA transporter, and EAAC1 of glutamic acid transporter that structurally has eight transmembrane domains. At first, this control model was studied based on the response of a glucose transporter to insulin, but, given the possible quick activity-dependent functional control, it is drawing much attention with regard to the neurological systems. The choline transporter is highly likely on this system, because it belongs in a glucose transporter family and shows extremely quick increase and decrease of choline transport at a synaptosome level. Such results suggest that mere incubation of the compound of the formula (I), particularly 4-acylamino-5,6,7,8-tetrahydrofuro[2,3-b]quinoline derivative, with hippocampal membrane samples would quickly increase the transporter number on the membrane. Membrane samples do not have a nucleus, and therefore, the transporter number cannot be increased by new protein synthesis. This leads to the conclusion that the increase in the transporter number on a cell membrane is derived from the pool in the cells. Thus, 4-acylamino-5,6,7,8-tetrahydrofuro[2,3-b]quinoline derivative is considered to have an action to draw out the transporters in the intracellular choline transporter store onto a nerve terminal, namely, have a point of action on the translocation of high-affinity choline transporters.

### Example 3: Sustention of action on high-affinity choline uptake

Because the compound of the formula (I), particularly 4-acylamino-5,6,7,8-tetrahydrofuro[2,3-b]quinoline derivative, has the above-mentioned mechanism, the promoting action on the high-affinity choline uptake is considered to be dependent on the density of high-affinity choline transporters on the nerve terminal, and therefore, to show a shift independent of the elimination rate of a drug. To clarify this point, the sustained promoting action on the high-affinity choline uptake was examined by monitoring the changes in efficacy when the aforementioned AF64A-treated rats were subjected to single and consecutive administration(s) of a drug. In the test, Compound A at each concentration or a solvent (vehicle) was administered singly or consecutively to AF64A-treated animals and the animals that underwent a sham operation. After a given time, the animals were slaughtered and hippocampal membrane samples (P2 fraction) were prepared by a conventional method. These membrane samples were incubated in Ringer's solution supplemented with [³H] choline at 37°C for 10 min. After the completion of the incubation, the membrane samples were filtrated on a glass fiber filter using a cell harvester and washed three times with a buffer. After thorough drying, a scintillator was added and the radioactivity of the membrane samples was measured on a liquid scintillation counter. The non-specific uptake was that in the presence of hemicholinium-3 (10 µM) in a sodium ion free Ringer's solution. To establish the close relationship between the efficacy observed here and an improving action on the dementia symptoms, the improving effect on the water maze-learning disorder observed in the AF64A-treated rats was also studied at a given time after drug administration for 8 consecutive days.

The water maze-learning disorder test was performed according to the method disclosed in JP-A-8-104633.

The results are shown in Fig. 4 - Fig. 7. The time-course HACU promoting action by single administration (Fig. 4) reveals a significant promotion of HACU at 1 and 3 h after administration. This efficacy attenuated 24 h later. In contrast, by the administration for 8 consecutive days (Fig. 5), a significant HACU promoting action was observed even after 24 h from the final administration. This effect was sustained until 48 h later and disappeared in 72 h. To examine the effect of consecutive administration, the efficacy at 24 h after administration for 4 consecutive days, 8 consecutive days and 16 consecutive days was also examined (Fig. 6), but significant HACU promotion was not observed after the administration for 4 consecutive days. By the administration for 16 consecutive days, the same level of improving effect as with the administration for 8 consecutive days was observed. In a behavioral test, too,. a significant improving effect on learning disorder was observed at 24 h after and 48 h after the administration for 8 consecutive days (Fig. 7).

It is apparent from this experiment that a high-affinity choline uptake promoting action by 4-acylamino-5,6,7,8-tetrahydrofuro[2,3-b]quinoline derivative is noticeably sustainable, as compared to the elimination time of a drug from the blood. The 4-acylamino-5,6,7,8-tetrahydrofuro[2,3-b]quinoline derivative leads out the transporters onto the nerve terminal, increases the choline transporter number on the nerve terminal, which decreased due to the disease state, and promotes high-affinity choline uptake. This action is considered to continue until the transporters are drawn out from the nerve terminal by translocation. In other words, the sustention of efficacy depends not on drug concentration in blood but metabolytic turnover of transporters. As a result, sustained efficacy can be expected even after disappearance of the pharmaceutical agent from the blood. By consecutive administration, moreover, the choline transporter number on the nerve terminal increases due to the accumulation of efficacy by single administration, whereby reinforcement of efficacy and sustention of the action are designed.

### Industrial Applicability

The present inventors have intensively studied the detailed action mechanism of the high-affinity choline uptake promoting action of 4-acylamino-5,6,7,8-tetrahydrofuro[2,3-b]quinoline derivative. As a result, it has been first elucidated that this pharmaceutical agent has an action of changing the intracellular locality of high-affinity choline transporters, thereby to promote transfer thereof onto the nerve terminal, unlike a known pharmaceutical agent having an acetylcholinergic nerve activating action. According to this action mechanism, a certain level of drug concentration is required for the expression of the action, but the efficacy once expressed shifts independently of disappearance of drug concentration, because it shifts depending on the physiological disappearance rate of the density of the high-affinity choline transporters on the nerve terminal.

This in turn means provision of a pharmaceutical agent that does not require administration frequency of at least 3 times a day in view of half life in blood, but permits striking administration frequency of not more than twice a day, preferably once a day, in consideration of the mechanism of efficacy. The decreased administration frequency itself means improved compliance of patients in need of the treatment with the pharmaceutical agent, and a reduced burden on the caregiver of the patient.

In the chronic diseases such as dementia, an overlooked dose of a pharmaceutical agent results in radical changes in disease conditions. An agent for the prophylaxis or therapy of cholinergic nerve disorder, which contains, as an active ingredient, a pharmaceutical agent, particularly 4-acylamino-5,6,7,8-tetrahydrofuro[2,3-b]quinoline derivative, having an action mechanism found in the present invention can avoid the risk associated with the drug administration itself, due to the property of the inventive agent. Even if medication is overlooked, interrupted administration of a pharmaceutical agent does not lead to a radical change in the disease state, because the density of the high-affinity choline transporters on the nerve terminal decreases gradually following the physiological disappearance rate.

The present invention provides a pharmaceutical agent based on the system wherein redundant transporters pooled in a nerve terminal is pulled out onto the nerve terminal to provide choline, which is a substrate of acetylcholine synthesis. The amount of the transporters utilizable for the promotion of choline uptake is limited in the body, and the pharmaceutical agent is based on the action mechanism for restoring the level of neuroactivity inherently possessed by the nerve but weakened by disease and disorder. Therefore, the side effects due to the excessive stimulation of the acetylcholinergic nerve, as can be seen with acetylcholine esterase inhibitors, are not expressed, thereby obliterating the need for a titration period.

This application is based on a patent application No. 238476/2001 filed in Japan.

## Claims

1. A pharmaceutical agent for increasing the choline transporter number on the cell membrane surface of a cholinergic nerve terminal, which agent comprises, as an active ingredient, a compound of the formula (I) 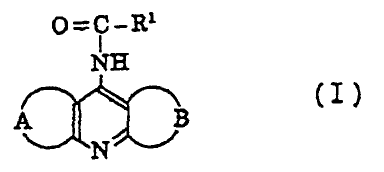 wherein R¹ is C₂-C₆ alkyl or the formula (II) 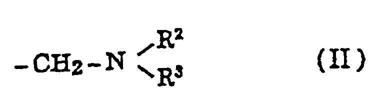 wherein R² is hydrogen atom or acetyl and R³ is C₁-C₆ alkyl, cycloalkyl or 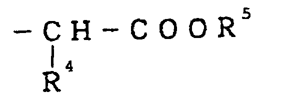 wherein R⁴ and R⁵ are each independently hydrogen atom or C₁-C₆ alkyl, and in 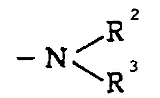 of the formula (II) , R² and R³ may in combination form 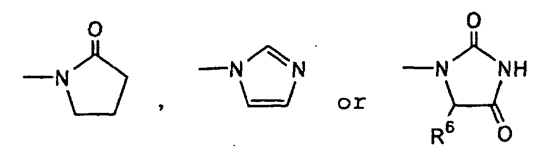 wherein R⁶ is hydrogen atom or C₁-C₆ alkyl; 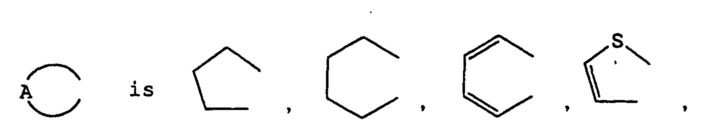 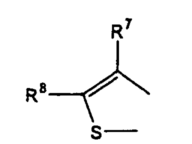 wherein R⁷ and R⁸ are each independently hydrogen atom or C₁-C₄ alkyl, 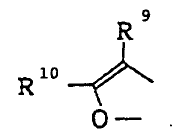 wherein R⁹ and R¹⁰ are each independently hydrogen atom or C₁-C₄ alkyl, 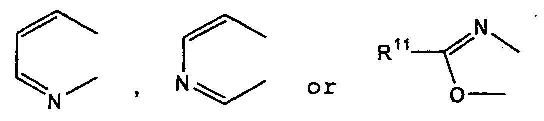 wherein R¹¹ is hydrogen atom or C₁-C₄ alkyl; and 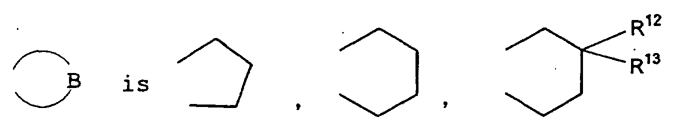 wherein R¹² and R¹³ are each independently C₁-C₄ alkyl or may in combination form 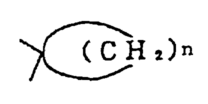 wherein n is an integer of 2 to 6, or 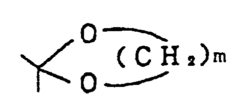 wherein m is an integer of 2 or 3, 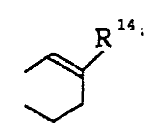 wherein R¹⁴ is hydrogen atom or C₁-C₄ alkyl, 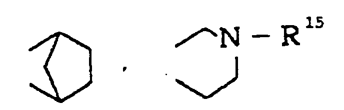 wherein R¹⁵ is hydrogen atom or aralkyl, or 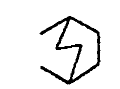 provided that when 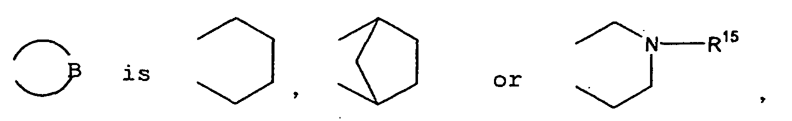 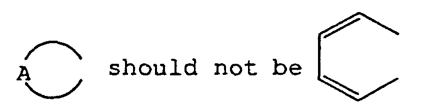 and R⁷ should not be hydrogen atom, optical enantiomers thereof, an acid addition salt thereof or a hydrate or solvate thereof.

2. The pharmaceutical agent of claim 1, wherein the compound of the formula (I) is a compound of the formula (Ia) 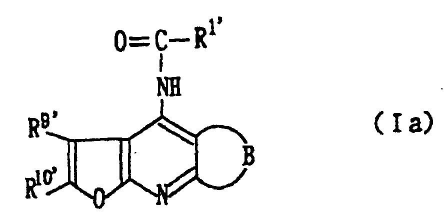 wherein R¹' is C₂-C₆ alkyl or the formula (II)' 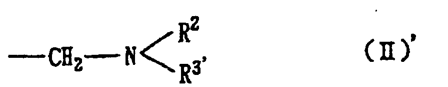 wherein R² is hydrogen atom or acetyl, and R³' is C₁-C₆alkyl or 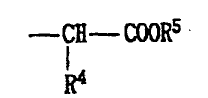 wherein R⁴ and R⁵ are each independently hydrogen atom or C₁-C₆ alkyl, and in 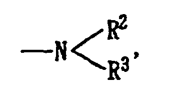 of the formula (II)', R² and R³' may in combination form 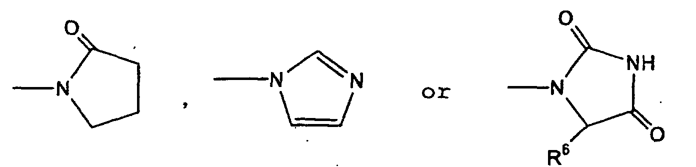 wherein R⁶ is hydrogen atom or C₁-C₆ alkyl;
R⁹' and R¹⁰' are each independently C₁-C₄ alkyl; and 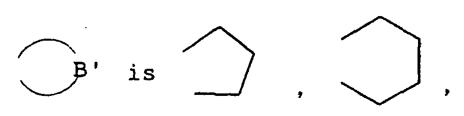 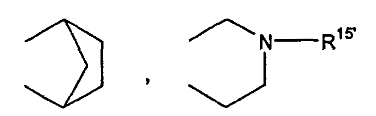 wherein R¹⁵' is aralkyl, or

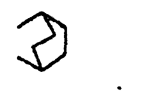

3. The pharmaceutical agent of claim 1, wherein the compound of the formula (I) is a 4-acylamino-5,6,7,8-tetrahydrofuro[2,3-b]quinoline derivative of the formula (Ib) 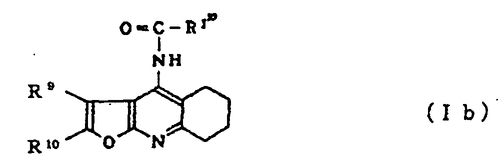 wherein R¹'' is C₂-C₆ alkyl, 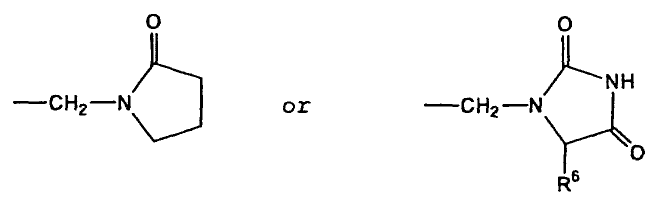 wherein R⁶ is hydrogen atom or C₁-C₆ alkyl; and
R⁹ and R¹⁰ are each independently hydrogen atom or C₁-C₄ alkyl.

4. The pharmaceutical agent of claim 1, wherein the compound of the formula (I) is 2-(2-oxopyrrolidin-1-yl)-N-(2,3-dimethyl-5,6,7,8-tetrahydrofuro[2,3-b]quinolin-4-yl)acetamide.

5. A pharmaceutical agent for increasing the choline transporter number on the cell membrane surface by acting on intracellular localized changes of the choline transporters, which agent comprises, as an active ingredient, a compound of the formula (I) of claim 1, optical enantiomers thereof, an acid addition salt thereof or a hydrate or solvate thereof.

6. The pharmaceutical agent of claim 5, wherein the compound of the formula (I) is a compound of the formula (Ia) of claim 2.

7. The pharmaceutical agent of claim 5, wherein the compound of the formula (I) is 4-acylamino-5,6,7,8-tetrahydrofuro[2,3-b]quinoline derivative represented by the formula (Ib) of claim 3.

8. The pharmaceutical agent of claim 5, wherein the compound of the formula (I) is 2-(2-oxopyrrolidin-1-yl)-N-(2,3-dimethyl-5,6,7,8-tetrahydrofuro[2,3-b]quinolin-4-yl)acetamide.

9. An agent for the prophylaxis or therapy of a cholinergic nerve disorder, which comprises, as an active ingredient, a pharmaceutical agent that increases the choline transporter number on the cell membrane surface of the cholinergic nerve terminal.

10. The prophylactic or therapeutic agent of claim 9, wherein the pharmaceutical agent is the agent of any of claims 1 to 4.

11. An agent for the prophylaxis or therapy of a cholinergic nerve disorder, which comprises, as an active ingredient, a pharmaceutical agent that acts on intracellular localized changes of choline transporters to increase the choline transporter number on the cell membrane surface.

12. The prophylactic or therapeutic agent of claim 11, wherein the pharmaceutical agent is the agent of any of claims 5 to 8.

13. The prophylactic or therapeutic agent of any of claims 9 to 12, wherein the cholinergic nerve disorder is dementia caused by Alzheimer's disease, Huntington's disease, Pick' disease, Down's syndrome or Parkinson's disease, tardive dyskinesia, myasthenia gravis, glaucoma or dysgryphia.

14. The prophylactic or therapeutic agent of any of claims 9 to 13, which is administered not more than twice a day.

15. The prophylactic or therapeutic agent of any of claims 9 to 14, which causes reduced side effects due to hyperstimulation of acetylcholinergic nerve.

16. A commercial package comprising a pharmaceutical agent of any of claims 1 to 4 and a written matter associated therewith, the written matter stating that the pharmaceutical agent can or should be used for the prophylaxis and/or therapy of a cholinergic nerve disorder.

17. A commercial package comprising a pharmaceutical agent of any of claims 5 to 8 and a written matter associated therewith, the written matter stating that the pharmaceutical agent can or should be used for the prophylaxis and/or therapy of a cholinergic nerve disorder.

18. The commercial package of claim 16 or 17, wherein the written matter states that the pharmaceutical agent is administered not more than twice a day.

19. A method of screening a compound useful for the prophylaxis and/or therapy of a cholinergic nerve disorder, which comprises at least the steps of
(1) bringing a test compound into contact with a cell membrane of a cholinergic nerve terminal, and
(2) counting the transporter number on a surface of the cell membrane.

20. A pharmaceutical agent for the prophylaxis or therapy of cholinergic nerve disorder, which comprises, as an active ingredient, a compound of the formula (I) described in claim 1, optical enantiomers thereof, an acid addition salt thereof or a hydrate or solvate thereof and which is administered not more than twice a day.

21. The prophylactic or therapeutic agent of claim 20, wherein the compound of the formula (I) is a compound of the formula (Ia) described in claim 2.

22. The prophylactic or therapeutic agent of claim 20, wherein the compound of the formula (I) is a 4-acylamino-5,6,7,8-tetrahydrofuro[2,3-b]quinoline derivative of the formula (Ib) described in claim 3.

23. The prophylactic or therapeutic agent of claim 20, wherein the compound of the formula (I) is 2-(2-oxopyrrolidin-1-yl)-N-(2,3-dimethyl-5,6,7,8-tetrahydrofuro[2,3-b]quinolin-4-yl)acetamide.

24. The prophylactic or therapeutic agent of any of claims 20 to 23, wherein the cholinergic nerve disorder is dementia caused by Alzheimer's disease, Huntington's disease, Pick' disease, Down's syndrome or Parkinson's disease, tardive dyskinesia, myasthenia gravis, glaucoma or dysgryphia.

25. The prophylactic or therapeutic agent of any of claims 20 to 23, wherein the cholinergic nerve disorder is dementia caused by Alzheimer's disease, tardive dyskinesia, myasthenia gravis, glaucoma or dysgryphia.

26. The prophylactic or therapeutic agent of any of claims 20 to 25, which is administered once a day.
